# EUROPÄISCHE PATENTSCHRIFT

(11) **EP 3 128 012 B1**
(45) Veröffentlichungstag und Bekanntmachung des Hinweises auf die Patenterteilung: **31.01.2018**
(21) Anmeldenummer: 15179762.8
(22) Anmeldetag: 05.08.2015
(51) Int. Cl.: C12P 13/00, C12P 13/04

(54) **VERFAHREN ZUR PEROXYGENASE-KATALYSIERTEN ABSPALTUNG VON BENZYLOXYCARBONYL-SCHUTZGRUPPEN**
PROCESS FOR PEROXYGENASE-CATALYZED CLEAVAGE OF BENZYLOXYCARBONYL PROTECTIVE GROUPS
PROCEDE DE SEPARATION DE PEROXYGENASE CATALYSEE DE GROUPES DE PROTECTION DE BENZYLOXYCARBONYLE

(43) Veröffentlichungstag der Anmeldung: 08.02.2017
(73) Patentinhaber: Sanofi-Aventis Deutschland GmbH, 65929 Frankfurt am Main (DE)
(72) Erfinder: Holla, Wolfgang, 65926 Frankfurt am Main (DE); Atzrodt, Jens, 65926 Frankfurt am Main (DE); Sandvoss, Martin, 65926 Frankfurt am Main (DE); Heidrich, Johannes, 65926 Frankfurt am Main (DE); Scheibner, Katrin, 03046 Cottbus (DE); Groebe, Glenn, 03046 Cottbus (DE); Kiebist, Jan, 03046 Cottbus (DE); Hofrichter, Martin, 02763 Zittau (DE); Poraj-Kobielska, Marzena, 02763 Zittau (DE)

(56) Entgegenhaltungen:
- DE-A1-102007 058 741
- US-B2- 7 416 876
- RAMESH&EMSP14;N. PATEL ET AL: "Enantioselective Enzymatic Cleavage of N-Benzyloxycarbonyl Groups", ADVANCED SYNTHESIS & CATALYSIS, Bd. 345, Nr. 67, Juni 2003 (2003-06), Seiten 830-834, XP055242152, DE ISSN: 1615-4150, DOI: 10.1002/adsc.200303038
- M. MAURS ET AL: "Microbial enantioselective removal of theN-benzyloxycarbonyl amino protecting group", JOURNAL OF MOLECULAR CATALYSIS. B, ENZYMATIC, Bd. 84, 2012, Seiten 22-26, XP028939176, ISSN: 1381-1177, DOI: 10.1016/J.MOLCATB.2012.03.005
- MARZENA PORAJ-KOBIELSKA ET AL: "Preparation of human drug metabolites using fungal peroxygenases", BIOCHEMICAL PHARMACOLOGY, ELSEVIER, US, Bd. 82, Nr. 7, 14. Juni 2011 (2011-06-14), Seiten 789-796, XP028263253, ISSN: 0006-2952, DOI: 10.1016/J.BCP.2011.06.020 [gefunden am 2011-06-23]

## Beschreibung

Die vorliegende Erfindung betrifft ein Verfahren zur Abspaltung von Benzyloxycarbonyl-Schutzgruppen, dadurch gekennzeichnet, dass eine N-Benzyloxycarbonyl-Verbindung oder N-(4-Methoxybenzyloxycarbonyl)-Verbindung in einem wasserhaltigen Lösungsmittel in Gegenwart einer unspezifischen Peroxygenase aus den Basidiomyceten Agrocybe aegerita, Coprinellus radians oder Marasmius rotula mit Wasserstoffperoxid umgesetzt wird.

Die Verwendung von Schutzgruppen, die vor Durchführung einer Reaktion in ein Molekül eingeführt und später wieder abgespalten werden, ist gängige Praxis bei der Synthese von organischen Verbindungen, wenn funktionelle Gruppen Anlass zu einem unerwünschten Reaktionsablauf oder zur Bildung von Nebenprodukten geben, und ist umfangreich beschrieben. Als Standardwerke zur Verwendung von Schutzgruppen seien genannt P. G. M. Wuts und T. W. Greene, Greene's Protective Groups in Organic Synthesis, 4. Auflage, Wiley, 2007; P. J. Kocienski, Protecting Groups, 3. Auflage, Thieme, 2003; und insbesondere zu Schutzgruppentechniken bei Peptidsynthesen M. Goodman et al. (Herausgeber) in Houben-Weyl, Methoden der Organischen Chemie (Methods of Organic Chemistry), Band E22a, Synthesis of Peptides and Peptidomimetics, Thieme, 2004.

Die wichtigste Klasse von Schutzgruppen für alle Formen von Aminen sind die sogenannten Carbamat-Schutzgruppen, die eine an ein Stickstoffatom gebundene Estergruppe -C(=O)-O-R enthalten, in der der Rest R vielfältige Bedeutungen haben kann. Sie werden beispielsweise zum temporären Schutz von Alkylaminen und Arylaminen, von N-haltigen Heterocyclen wie Imidazolen, Pyrrolen und Indolen, und insbesondere von Aminosäuren und Peptiden verwendet, und können durch Umsetzung mit geeigneten Kohlensäureester-Derivaten in das Molekül eingeführt werden und später mit geeigneten Reagenzien wieder abgespalten werden. Zu den besonders nützlichen Carbamat-Schutzgruppen gehören die tert-Butyloxycarbonylgruppe (Boc; R = tert-Butyl), die durch Einwirkung von Säuren abgespalten werden kann, die Benzyloxycarbonylgruppe (Z oder Cbz; R = Benzyl), die durch katalytische Hydrierung abgespalten werden kann, die 9-Fluorenylmethyloxycarbonylgruppe (Fmoc; R = 9-Fluorenylmethyl), die durch basische beta-Eliminierung beispielsweise durch Einwirkung von Piperidin abgespalten werden kann, und die Allyloxycarbonylgruppe (Aloc; R = Allyl), die durch Metall-katalysierte Deallylierung in Gegenwart eines nucleophilen Allylfängers abgespalten werden kann.

Die zuerst von M. Bergmann et al., Ber. Dt. Chem. Ges. 1932, 65, 1192 beschriebene Benzyloxycarbonyl-Schutzgruppe ist eine der meist genutzten Schutzgruppen. Ihre häufige Verwendung ist zurückzuführen auf die einfache Abspaltung durch Hydrierung und die dadurch erreichte Orthogonalität zu vielen anderen Schutzgruppen einschließlich der häufig verwendeten Gruppen Boc und Fmoc, d. h. die Möglichkeit, die Abspaltung mit Reagenzien bzw. unter Reaktionsbedingungen durchzuführen, die die anderen Schutzgruppen nicht beeinträchtigen bzw. abspalten, und die Abspaltung der verschiedenen Schutzgruppen in beliebiger Reihenfolge durchzuführen. Neben der unsubstituierten Benzyloxycarbonylgruppe werden auch verschiedene in der Phenylgruppe substituierte Benzyloxycarbonylgruppen mit anderen Eigenschaften und Reaktivitäten als Schutzgruppen verwendet, zum Beispiel die 4-Methoxybenzyloxycarbonylgruppe und die 3,5-Di-tert-butylbenzyloxycarbonylgruppe. Zur Einführung der Benzyloxycarbonyl-Schutzgruppen gibt es eine Reihe von Methoden, die zum Beispiel in den erwähnten Standardwerken beschrieben sind. Viele basieren auf der Umsetzung der zu schützenden Verbindung mit Chlorkohlensäurebenzylestern (Chlorameisensäurebenzylestern) bzw. chemisch ähnlichen Verbindungen.

Auch zur Abspaltung der Benzyloxycarbonyl-Schutzgruppen gibt es eine Reihe von Methoden. Als Beispiele seien genannt die oben erwähnte Hydrierung unter unterschiedlichen Bedingungen, zum Beispiel mit Wasserstoff in Gegenwart eines Katalysators wie Palladium auf Kohle, Reduktionen unter Verwendung gelöster Metalle, zum Beispiel mit Lithium oder Natrium in flüssigem Ammoniak, Acidolysen, zum Beispiel mit Salzsäure unter Rückfluss oder mit Bromwasserstoff in Essigsäure, oder insbesondere bei Peptidsynthesen die Einwirkung von Fluorwasserstoff in Gegenwart von Dimethylsulfid und p-Kresol. Auch Abspaltungen unter basischen Bedingungen, zum Beispiel mit Kaliumhydroxid in Wasser/Methanol, wurden beschrieben. Darüber hinaus gibt es weitere reduktive Methoden und auch elektrolytische, photolytische und biotechnologische Methoden zur Abspaltung von Benzyloxycarbonyl-Schutzgruppen.

Zu den biotechnologischen Methoden gehören beispielsweise die Anwendung isolierter Enzyme wie der Penicillin G-Acylase (G. Alvaro et al., Biocatal. Biotransform. 2000, 18, 253) und von Urethan-Hydrolasen (V. B. Nanduri et al., Enzyme Microb. Technol. 2004, 34, 304; E. Matsumura et al., Agric. Biol. Chem. 1985, 49, 973) sowie die Anwendung von Zellextrakten aus dem Mikroorganismus Sphingomonas paucimobilis SC 16113 (R. N. Patel et al., Adv. Synth. Catal. 2003, 345, 830). In M. Maurs et al., J. Mol. Cat. B: Enzymatic 2012, 84, 22 wird die Anwendung von Ganzzell-Präparaten von Arthrobacter-Stämmen für die enantioselektive Hydrolyse von N-Benzyloxycarbonyl-geschützten Aminosäuren beschrieben, bei der zum Beispiel aus racemischem N-Benzyloxycarbonyl-Allylglycin L-Allylglycin mit einer optischen Reinheit von > 98 % und N-Benzyloxycarbonyl-D-Allylglycin erhalten wurden. Die beschriebene Methode ermöglicht die EnantiomerenTrennung von Benzyloxycarbonyl-geschützten Aminosäuren mit freier α-Carbonsäure-Funktion, nicht aber die Abspaltung der N-Benzyloxycarbonyl-Schutzgruppe von Aminosäureestern und Aminosäureamiden.

Trotz der Vielzahl und der Unterschiedlichkeit der beschriebenen Methoden gelingt die Abspaltung von Benzyloxycarbonyl-Schutzgruppen aber nicht immer mit der erwünschten hohen Ausbeute und Chemoselektivität. So können beispielsweise bei der gebräuchlichen hydrogenolytischen Abspaltung von Benzyloxycarbonylgruppen andere im Molekül vorhandene funktionelle Gruppen, zum Beispiel Alkenylgruppen und Alkinylgruppen mit ihren Mehrfachbindungen, Benzylethergruppen und substituierte Benzylethergruppen wie Nitrobenzylethergruppen und Methoxybenzylethergruppen, Carbonsäurebenzylestergruppen, Nitrogruppen und schwefelhaltige Gruppen, zu unerwünschten Nebenprodukten durch gleichzeitige Hydrierung bzw. Abspaltung der Benzylgruppen und Komplikationen wie einer Vergiftung des Katalysators führen. Die Anwendung drastischer reduktiver, saurer oder basischer Bedingungen in Gegenwart labiler Gruppen oder bei zur Racemisierung tendierenden Verbindungen ist ebenfalls oft ungeeignet. Die bisher beschriebenen Methoden der enzymatischen oder mikrobiellen Hydrolyse von Benzyloxycarbonyl-Schutzgruppen können bei der Anwendung auf Verbindungen mit Estergruppen oder N-Acylgruppen zu unerwünschten Hydrolyseprodukten führen. Es besteht daher Bedarf an weiteren Methoden für die Abspaltung von Benzyloxycarbonyl-Schutzgruppen mit geeigneten Chemoselektivitätsmustern für die mannigfaltigen Probleme der organischen Synthese.

Es wurde nun gefunden, dass Benzyloxycarbonylgruppen an einem Stickstoffatom chemoselektiv, bei gleichzeitiger Stabilität verschiedener anderer funktioneller Gruppen, zum Beispiel von Mehrfachbindungen, Carbonsäureestergruppen einschließlich Carbonsäurebenzylestergruppen, und unsubstituierten und substituierten Benzylethergruppen wie Chlorbenzyl-, Cyanbenzyl-, Methoxybenzyl-, Methylbenzyl- und Nitrobenzylethergruppen, abgespalten werden können durch Umsetzung von N-Benzyloxycarbonyl-Verbindungen, d. h. Verbindungen, die an einem Stickstoffatom eine unsubstituierte Benzyloxycarbonylgruppe tragen, und N-(4-Methoxybenzyloxycarbonyl)-Verbindungen mit Wasserstoffperoxid in Gegenwart von Peroxygenasen aus Basidiomyceten, also Enzymen (Biokatalysatoren), die sich mechanistisch von den oben erwähnten Enzymen wie Hydrolasen grundsätzlich unterscheiden.

Gegenstand der vorliegenden Anmeldung ist ein Verfahren zur Abspaltung von Benzyloxycarbonyl-Schutzgruppen, dadurch gekennzeichnet, dass eine N-Benzyloxycarbonyl-Verbindung oder N-(4-Methoxybenzyloxycarbonyl)-Verbindung in einem wasserhaltigen Lösungsmittel in Gegenwart einer unspezifischen Peroxygenase aus den Basidiomyceten Agrocybe aegerita, Coprinellus radians oder Marasmius rotula mit Wasserstoffperoxid umgesetzt wird.

Gegenstand der Erfindung ist auch die Verwendung einer unspezifischen Peroxygenase aus den Basidiomyceten Agrocybe aegerita, Coprinellus radians oder Marasmius rotula zur Abspaltung von Benzyloxycarbonyl-Schutzgruppen durch Umsetzung einer N-Benzyloxycarbonyl-Verbindung oder einer N-(4-Methoxybenzyloxycarbonyl)-Verbindung mit Wasserstoffperoxid in einem wasserhaltigen Lösungsmittel. Alle Ausführungen zum erfindungsgemäßen Verfahren gelten entsprechend für die erfindungsgemäße Verwendung.

Die Erfindung betrifft somit die Umwandlung einer Verbindung der Formel I in die Verbindung der Formel II, 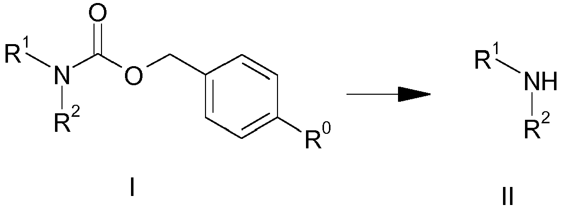 in denen R⁰ für Wasserstoff oder Methoxy (CH₃O) steht und R¹ und R² vielfältige Bedeutungen haben können, insbesondere auch eine der beiden Gruppen R¹ und R² für Wasserstoff stehen kann und in einer Ausführungsform der Erfindung eine der beiden Gruppen R¹ und R² für Wasserstoff steht. In den Verbindungen der Formeln I und II können die beiden Gruppen R¹ und R² zusammen mit dem sie tragenden Stickstoffatom auch einen Ring bilden. Die eingesetzte Verbindung der Formel I, also das Substrat der erfindungsgemäßen Enzymreaktion, kann beispielsweise eine an einem Stickstoffatom durch eine Benzyloxycarbonylgruppe oder 4-Methoxybenzyloxycarbonylgruppe geschützte Verbindung aus den Klassen der acyclischen und cyclischen Amine, Aminoalkohole, Aminoether, Aminosäurederivate einschließlich Aminosäuren, Aminosäureestern und Aminosäureamiden, und Peptidderivate einschließlich Dipeptid-, Tripeptid- und Polypeptidderivaten und einschließlich Peptiden, Peptidestern und Peptidamiden sein, und das Produkt der Formel II dann die entsprechende ungeschützte Verbindung aus diesen Klassen. Im Fall von Aminosäuren, Aminosäureestern, Aminosäureamiden, Peptiden, Peptidestern und Peptidamiden kann somit beispielsweise R¹ in den Formeln I und II für den Rest stehen, der formal aus einer Aminosäure, einem Aminosäureester, einem Aminosäureamid, einem Peptid, einem Peptidester oder einem Peptidamid durch Entfernen der Aminogruppe erhalten wird, und R² beispielsweise für Wasserstoff stehen. In einer Ausführungsform der Erfindung steht R⁰ für Wasserstoff und betrifft die Erfindung somit die Abspaltung von unsubstituierten Benzyloxycarbonylgruppen, in einer weiteren Ausführungsform steht R⁰ für Methoxy und betrifft die Erfindung somit die Abspaltung von 4-Methoxybenzyloxycarbonylgruppen.

Das erfindungsgemäße Verfahren kann nach dem Fachmann geläufigen Standardprozeduren für enzymkatalysierte Reaktionen durchgeführt werden, indem das Substrat der Formel I, das Wasserstoffperoxid, die Peroxygenase aus Basidiomyceten und gegebenenfalls Hilfsstoffe in geeigneter Weise in einem wasserhaltigen Lösungsmittel vereint werden und das Reaktionsgemisch nach Ablauf der Reaktion nach Standardmethoden aufgearbeitet wird, wobei wie üblich die zweckmäßige Vorgehensweise im Detail vom Fachmann nach den Gegebenheiten des Einzelfalles gewählt wird.

Bei den verwendeten Peroxygenasen aus Basidiomyceten handelt es sich um extrazelluläre Häm-Thiolat-Proteine, die Eigenschaften von klassischen Peroxidasen und Monooxygenasen in sich vereinen und daher als Hybridenzyme verstanden werden können (M. Hofrichter et al., Curr. Opin. Chem. Biol. 2014, 19, 116; M. Hofrichter et al., Appl. Microbiol. Biotechnol. 2010, 87, 871; M. Hofrichter et al., Appl. Microbiol. Biotechnol. 2006, 71, 276). Zur Enzym-Subklasse der Peroxygenasen (Enzymklassifikation EC 1.11.2) gehören die unspezifische Peroxygenase, die Myeloperoxidase, die Pflanzensamenperoxygenase und die Fettsäureperoxygenase. Eine Peroxygenase des Typs, der heute allgemein als unspezifische Peroxygenase (UPO; Enzymklassifikation EC 1.11.2.1) bezeichnet wird und vormals als Haloperoxidase oder als aromatische Peroxygenase (APO) bezeichnet wurde, wurde erstmals aus der Basidiomycete Agrocybe aegerita (Südlicher Ackerling) isoliert. Ähnliche Enzymaktivitäten wurden unter anderem in den Kulturmedien der Basidiomyceten Coprinellus radians (teilweise bezeichnet als Coprinus radians) und Marasmius rotula nachgewiesen. Bei den unspezifischen Peroxygenasen handelt es sich um stark glycosylierte Proteine mit Molekularmassen von 32 bis 46 kDa und isoelektrischen Punkten zwischen pl 3.5 und pl 6.0. In Abhängigkeit vom Enzym, Substrat und den Reaktionsbedingungen katalysieren die unspezifischen Peroxygenasen eine Vielzahl von Reaktionen wie Halogenierungen, Oxygenierungen und Oxidationen durch Ein-Elektronen-Abstraktion. Die Enzyme benötigen keine Cofaktoren wie NADP(H), sondern lediglich Wasserstoffperoxid, wobei ein Sauerstoffatom des Peroxids auf das Substrat übertragen wird. Mechanistisch kann die Katalyse als Analogie zum H₂O₂-shunt pathway der Cytochrom P450-Monooxygenasen angesehen werden.

Die Isolierung und Reinigung der verwendeten extrazellulären Peroxygenasen aus Basidiomyceten ist in der Literatur beschrieben. Die Gewinnung der Peroxygenase aus Agrocybe aegerita (AaeUPO) ist beschrieben in R. Ullrich et al., Appl. Environ. Microbiol. 2004, 70, 4575, und K. Piontek et al., Acta Cryst. Sect. F 2010, F66, 693. Die Gewinnung der Peroxygenase aus Coprinellus radians (CraUPO) ist beschrieben in D. H. Anh et al., Appl. Environ. Microbiol. 2007, 73, 5477. Die Gewinnung der Peroxygenase aus Marasmius rotula (MroUPO) ist beschrieben in G. Gröbe et al., AMB Express 2011, 1, 31.Die verwendete Peroxygenase aus Basidiomyceten ist eine unspezifische Peroxygenase (Enzymklassifikation EC 1.11.2.1) aus Agrocybe aegerita, Coprinellus radians oder Marasmius rotula, zum Beispiel eine unspezifische Peroxygenase aus Agrocybe aegerita oder eine unspezifische Peroxygenase aus Coprinellus radians oder eine unspezifische Peroxygenase aus Marasmius rotula.

Die Peroxygenase kann in fester Form, zum Beispiel lyophilisiert, in gelöster Form, zum Beispiel in wässriger, gegebenenfalls gepufferter und/oder mit anderen Hilfsstoffen versetzter Lösung, oder in immobilisierter Form, zum Beispiel auf einem Träger oder in einem Polyvinylalkohol-Polyethylenglykol-Gel verkapselt (M. Poraj-Kobielska et al., Biochem. Eng. J. 2015, 98, 144; siehe auch W. Hartmeier, Immobilized Biocatalysts, Springer, 1988), eingesetzt werden. Die Enzymmenge wird in Abhängigkeit von der Art des Enzyms und der Geschwindigkeit der durchzuführenden Reaktion bzw. von der angestrebten Reaktionszeit, von der eingesetzten Form und von anderen Parametern wie der Temperatur gewählt und kann durch Vorversuche leicht ermittelt werden. Im Allgemeinen wird pro mmol Substrat der Formel I das Enzym in einer Menge von ca. 100 U bis ca. 10000 U, in einer Ausführungsform von ca. 100 U bis ca. 5000 U, in einer weiteren Ausführungsform von ca. 500 U bis ca. 5000 U, eingesetzt, wobei eine Enzymeinheit U (unit) im Fall der Peroxygenasen aus Basidiomyceten gemäß der Definition in der Literatur die Enzymmenge ist, die 1 µmol Veratrylalkohol (3,4-Dimethoxybenzylakohol) in 1 Minute bei 23 °C und pH 7 zu 3,4-Dimethoxybenzaldehyd oxidiert (M. Poraj-Kobielska et al., Biochem. Eng. J. 2015, 98, 144; R. Ullrich et al., Appl. Environ. Microbiol. 2004, 70, 4575).

Das Wasserstoffperoxid kann in Form einer Lösung eingesetzt werden, zum Beispiel in Form einer wässrigen Lösung mit einem Gehalt von ca. 0.1 Gewichts-% bis ca. 35 Gewichts-% Wasserstoffperoxid, in einer Ausführungsform in Form einer wässrigen Lösung mit einem Gehalt von ca. 0.1 Gewichts-% bis ca. 5 Gewichts-%, zum Beispiel in Form einer wässrigen Lösung mit einem Gehalt von ca. 3 Gewichts-%, wobei die zweckmäßige Konzentration wie üblich unter anderem von der Ansatzgröße abhängt und bei größeren Ansätzen eher eine konzentriertere Lösung, bei kleineren Ansätzen eher eine verdünntere Lösung eingesetzt wird. Das Wasserstoffperoxid kann auch in anderer Form eingesetzt werden, zum Beispiel in Form des festen, als Pulver oder Tablette kommerziell erhältlichen 1:1-Addukts aus Harnstoff und Wasserstoffperoxid, oder es kann in dem Reaktionsgemisch durch einen chemischen oder biochemischen Prozess in situ erzeugt werden, zum Beispiel aus Glucose und Sauerstoff mit Hilfe der Glucose-Oxidase (EC 1.1.3.4). Das Wasserstoffperoxid wird im Allgemeinen im Überschuss eingesetzt, zum Beispiel in der ca. 1-fachen bis ca. 100-fachen, in einer Ausführungsform in der ca. 1-fachen bis ca. 50-fachen, in einer weiteren Ausführungsform in der ca. 5-fachen bis ca. 50-fachen, in einer weiteren Ausführungsform in der ca. 5-fachen bis ca. 20-fachen, molaren Menge, bezogen auf das Substrat der Formel I. Bei der Durchführung des erfindungsgemäßen Verfahrens kann die Wasserstoffperoxid-Konzentration im Reaktionsgemisch zum Beispiel bei ca. 0.1 mM (mMol/l) bis ca. 20 mM, in einer Ausführungsform bei ca. 0.1 mM bis ca. 10 mM, in einer weiteren Ausführungsform bei ca. 0.5 mM bis ca. 10 mM, in einer weiteren Ausführungsform bei ca. 1 mM bis ca. 5 mM, liegen, hängt aber natürlich von der Verfahrensführung ab und kann sich während der Durchführung der Reaktion verändern. In einer Ausführungsform werden das Substrat, die Peroxygenase und gegebenenfalls Hilfsstoffe im wasserhaltigen Lösungsmittel vorgelegt und wird unter Durchmischung des Reaktionsgemisches, zum Beispiel durch Rühren, das Wasserstoffperoxid sukzessive, oder nach und nach, über einen Zeitraum von mehreren Stunden, zum Beispiel über ca. 2 Stunden bis ca. 24 Stunden, in einer Ausführungsform über ca. 2 Stunden bis ca. 12 Stunden, in einer weiteren Ausführungsform über ca. 2 Stunden bis ca. 4 Stunden, zudosiert, zum Beispiel in Portionen oder kontinuierlich, bei Verwendung einer Wasserstoffperoxidlösung zum Beispiel mit Hilfe einer Pumpe, und gegebenenfalls werden auch Peroxygenase und/oder Hilfsstoffe nachdosiert. In einer weiteren Ausführungsform werden das Substrat, die Peroxygenase, das Wasserstoffperoxid und gegebenenfalls Hilfsstoffe im wasserhaltigen Lösungsmittel vorgelegt und das Reaktionsgemisch wird durchmischt, zum Beispiel gerührt, gegebenenfalls unter Nachdosieren von Peroxygenase und/oder Wasserstoffperoxid und/oder Hilfsstoffen.

Das wasserhaltige, oder wässrige, Lösungsmittel, in dem das erfindungsgemäße Verfahren durchgeführt wird, kann Wasser sein oder ein Gemisch aus Wasser und einem oder mehreren organischen Lösungsmitteln, durch die als Cosolventien die Löslichkeit des Substrats der Formel I im Reaktionsgemisch erhöht wird und die uneingeschränkt mit Wasser mischbar sein können oder nicht uneingeschränkt mit Wasser mischbar sein können. In einer Ausführungsform wird das Verfahren in einem Gemisch aus Wasser und einem oder mehreren organischen Lösungsmitteln durchgeführt, in einer weiteren Ausführungsform in einem Gemisch aus Wasser und einem oder mehreren uneingeschränkt mit Wasser mischbaren organischen Lösungsmitteln. Die geeignete Menge bzw. Konzentration an zugesetztem organischen Lösungsmittel hängt zum Beispiel von der Löslichkeit des Substrats der Formel I ab und kann sich während der Durchführung der Reaktion auch verändern, zum Beispiel durch Zudosieren einer wässrigen Lösung von Wasserstoffperoxid, und beträgt im Allgemeinen ca. 0.5 Volumen-% bis ca. 50 Volumen-%, in einer Ausführungsform ca. 0.5 Volumen-% bis ca. 30 Volumen-%, in einer weiteren Ausführungsform ca. 1 Volumen-% bis ca. 20 Volumen-% organisches Lösungsmittel, bezogen auf das gesamte wasserhaltige Lösungsmittel. Geeignete organische Lösungsmittel sind zum Beispiel Alkohole, etwa (C₁-C₄)-Alkanole wie Methanol, Ethanol, tert-Butanol, Ketone, etwa (C₃-C₅)-Alkanone wie Aceton, Nitrile wie Acetonitril, chlorierte Kohlenwasserstoffe wie Dichlormethan, Amide wie Dimethylformamid (DMF), Sulfoxide wie Dimethylsulfoxid (DMSO), Ether wie 1,2-Dimethoxyethan (DME), Tetrahydrofuran (THF), Dioxan, und Ester wie Essigsäureethylester. In einer Ausführungsform der Erfindung wird das Substrat der Formel I in einer geeigneten Menge organischem Lösungsmittel oder einer Mischung aus organischem Lösungsmittel und einem geringen Anteil Wasser gelöst und die Lösung mit Wasser, Peroxygenase, Wasserstoffperoxid und gegebenenfalls Hilfsstoffen versetzt. Je nach den Gegebenheiten des Einzelfalles kann die Reaktionsmischung das Substrat anfänglich zum Beispiel in einer Konzentration von ca. 0.1 Gewichts-% bis ca. 50 Gewichts-%, in einer Ausführungsform von ca. 0.1 Gewichts-% bis ca. 10 Gewichts-%, enthalten.

Das erfindungsgemäße Verfahren wird im Allgemeinen bei einem pH-Wert von ca. 4 bis ca. 9, in einer Ausführungsform bei einem pH-Wert von ca. 5 bis ca. 8, in einer weiteren Ausführungsform bei einem pH-Wert von ca. 5 bis ca. 7.5, in einer weiteren Ausführungsform bei einem pH-Wert von ca. 5.5 bis ca. 7.5 durchgeführt. Der pH-Wert des Reaktionsgemisches, der durch saure und basische Gruppen im Substrat der Formel I und dem Produkt der Formel II beeinflusst wird und sich im Laufe der Reaktion ändern kann, kann durch Zudosieren von Säuren, zum Beispiel Salzsäure, oder von Basen, zum Beispiel Natronlauge, im gewünschten Bereich gehalten werden oder durch Zugabe von Puffersubstanzen bzw. Pufferlösungen eingestellt werden. Säuren, Basen, Puffersubstanzen und Pufferlösungen sind Beispiele für Hilfsstoffe, die bei der Durchführung des erfindungsgemäßen Verfahrens zugegeben werden können. In einer Ausführungsform wird die Reaktion in Gegenwart eines Puffers durchgeführt, also unter Zugabe von Puffersubstanzen oder Pufferlösungen. Zur Pufferung des Reaktionsgemisches können übliche Puffer verwandt werden, zum Beispiel Phosphat-Puffer, Tris-Puffer (Tris-(hydroxymethyl)-methylamin-Puffer) oder der Phosphat und Citronensäure enthaltende Mcllvaine-Puffer. Die Konzentration an zugesetzter Puffersubstanz im Reaktionsgemisch kann zum Beispiel bei ca. 0.01 M bis ca. 1 M, in einer Ausführungsform bei ca. 0.01 M bis ca. 0.5 M, liegen.

Ein weiteres Beispiel für Hilfsstoffe, die bei der Durchführung des erfindungsgemäßen Verfahrens zugegeben werden können, sind Detergentien, oder oberflächenaktive Stoffe, Netzmittel, Lösungsvermittler oder Emulgatoren, die mit biologischen Substanzen verträglich sind oder nicht denaturierend sind und auch sonst bei Arbeiten mit biologischen Substanzen Verwendung finden, zum Beispiel zwitterionische Detergentien wie etwa CHAPS (3-[(3-Cholamidopropyl)dimethylammonio]-1 -propansulfonat) oder nichtionische Detergentien wie Triton X-100 (Polyethylenglycol-(p-(1,1,3,3-tetramethylbutyl)-phenyl)-ether, 4-Octylphenol-polyethoxylat) und Tween 80 (Polysorbat 80, Polyoxyethylen (20)-sorbitan-monooleat). Die Konzentration an zugesetztem Detergens im Reaktionsgemisch kann zum Beispiel bei ca. 0.01 Gewichts-% bis ca. 0.5 Gewichts-%, in einer Ausführungsform bei ca. 0.02 Gewichts-% bis ca. 0.2 Gewichts-%, in einer weiteren Ausführungsform bei ca. 0.05 Gewichts-% bis ca. 0.2 Gewichts-%, bezogen auf das gesamte Reaktionsgemisch, liegen.

Ein weiteres Beispiel für Hilfsstoffe, die bei der Durchführung des erfindungsgemäßen Verfahrens zugegeben werden können, sind Radikalfänger, zum Beispiel Ascorbinsäure, deren Gegenwart bei der Durchführung des Verfahrens einen stabilisierenden Effekt haben kann, insbesondere bei der Umsetzung von Verbindungen der Formel I, die aromatische Gruppen enthalten (siehe A. Karich et al., AMB Express 2013, 3, 5). Die Konzentration an zugesetztem Radikalfänger, beispielsweise Ascorbinsäure, im Reaktionsgemisch kann zum Beispiel bei ca. 0.01 mM bis ca. 100 mM, in einer Ausführungsform bei ca. 0.1 mM bis ca. 10 mM, in einer weiteren Ausführungsform bei ca. 0.5 mM bis ca. 5 mM, liegen. Ein Radikalfänger, beispielsweise Ascorbinsäure, kann zu Beginn der Reaktion dem Reaktionsgemisch zugesetzt werden und kann auch während des Ablaufs der Reaktion sukzessive, zum Beispiel portionsweise oder kontinuierlich, zudosiert werden, zum Beispiel in Form einer wässrigen Lösung.

Die Reaktionstemperatur bei der Durchführung des erfindungsgemäßen Verfahrens liegt im Allgemeinen bei ca. 10 °C bis ca. 70 °C, in einer Ausführungsform bei ca. 15 °C bis ca. 55 °C, in einer weiteren Ausführungsform bei ca. 20 °C bis ca. 45 °C, beispielsweise bei Raumtemperatur. Die Temperatur kann auch variiert werden und zum Beispiel im Laufe der Durchführung der Reaktion zur Vervollständigung der Umsetzung erhöht werden. Das erfindungsgemäße Verfahren kann auch unter Behandlung des Reaktionsgemisches mit Ultraschall durchgeführt werden.

### Beispiele

**Abkürzungen**

| | |
|---|---|
| 2NMC | 2-Naphthylmethyloxycarbonyl |
| 4CIZ | 4-Chlorbenzyloxycarbonyl |
| 4MeOZ | 4-Methoxybenzyloxycarbonyl |
| 4NO₂Bn | 4-Nitrobenzyl |
| 4NO₂Z | 4-Nitrobenzyloxycarbonyl |
| AaeUPO | Peroxygenase aus Agrocybe aegerita |
| Ala | Alanyl |
| Aloc | Allyloxycarbonyl |
| Arg | Arginyl |
| Asn | Asparaginyl |
| Asp | Aspartyl |
| Boc | tert-Butyloxycarbonyl |
| Bom | Benzyloxymethyl |
| tBu | tert-Butyl |
| CraUPO | Peroxygenase aus Coprinellus radians |
| Fmoc | 9-Fluorenylmethyloxycarbonyl |
| His | Histidyl |
| Me | Methyl |
| MroUPO | Peroxygenase aus Marasmius rotula |
| Mtr | 4-Methoxy-2,3,6-trimethylbenzolsulfonyl |
| Phe | Phenylalanyl |
| Ser | Seryl |
| Thr | Threonyl |
| Tos | Tosyl |
| Tyr | Tyrosyl |
| U | Enzymaktivitäts-Einheit |
| Xant | 9-Xanthyl |
| Z | Benzyloxycarbonyl |

Die Verbindungen sind in der in der Aminosäure- und Peptidchemie gebräuchlichen Weise bezeichnet. Beispielsweise steht Z-L-His(Bom)-OH für L-Histidin, das an der Aminogruppe eine Benzyloxycarbonylgruppe trägt, an der Carbonsäuregruppe als freie Säure vorliegt und in der Seitenkette am Stickstoffatom im Imidazolring eine Benzyloxymethylgruppe trägt.

Zur Herstellung der verwendeten Phosphat-Citronensäure-Puffer (Mcllvaine-Puffer) wurden eine 0.2 M Dinatriumhydrogenphosphatlösung durch Auflösen von 28.39 g wasserfreiem Dinatriumhydrogenphosphat in 1 l bidestilliertem Wasser und eine 0.1 M Citronensäurelösung durch Auflösen von 19.21 g wasserfreier Citronensäure in 1 l bidestilliertem Wasser angesetzt. Für 100 ml des Puffers mit pH 7 wurden 82.4 ml der Dinatriumhydrogenphosphatlösung und 17.6 ml der Citronensäurelösung zusammengeben, für 100 ml des Puffers mit pH 5.5 wurden 56.9 ml der Dinatriumhydrogenphosphatlösung und 43.1 ml der Citronensäurelösung zusammengegeben. Der pH-Wert wurde mit einer pH-Elektrode kontrolliert.

Die verwendete Peroxygenase aus Agrocybe aegerita (AaeUPO; siehe R. Ullrich et al., Appl. Environ. Microbiol. 2004, 70, 4575) wurde in Form einer Lösung mit einer Konzentration von 100 U/ml in Phosphat-Citronensäure-Puffer mit pH 7 eingesetzt. Die verwendete Peroxygenase aus Coprinellus radians (CraUPO; siehe D. H. Anh et al., Appl. Environ. Microbiol. 2007, 73, 5477) wurde in Form einer Lösung mit einer Konzentration von 33.6 U/ml bzw. 75 U/ml in Phosphat-Citronensäure-Puffer mit pH 7 eingesetzt. Die verwendete Peroxygenase aus Marasmius rotula (MroUPO; siehe G. Gröbe et al., AMB Express 2011, 1, 31) wurde in Form einer Lösung mit einer Konzentration von 100 U/ml in Phosphat-Citronensäure-Puffer mit pH 5.5 eingesetzt. Die Lösungen wurden durch Verdünnen stärker konzentrierter Stammlösungen mit Phosphat-Citronensäure-Puffer bzw. im Fall von lyophilisierten Enzymen durch Auflösen in Wasser, Zentrifugieren und Verdünnen des Überstands mit Phosphat-Citronensäure-Puffer hergestellt. Die Bestimmung der Enzymaktivität (in U) erfolgte wie in der Literatur beschrieben (siehe M. Poraj-Kobielska et al., Biochem. Eng. J. 2015, 98, 144; R. Ullrich et al., Appl. Environ. Microbiol. 2004, 70, 4575).

In den Beispielen 1 bis 29 wurde die Abspaltung der Z-Schutzgruppe aus verschiedenen Substraten unter standardisierten Bedingungen untersucht. 500 µl Phosphat-Citronensäure-Puffer mit pH 7 im Fall der Peroxygenasen AaeUPO und CraUPO bzw. mit pH 5.5 im Fall der Peroxygenase MroUPO, 275 µl bidestilliertes Wasser, 25 µl einer 40 mM Ascorbinsäurelösung in Wasser, 30 µl der Peroxygenaselösung mit einer Konzentration von 100 U/ml im Fall der Peroxygenasen AaeUPO und MroUPO bzw. 33.6 U/ml im Fall der Peroxygenase CraUPO in Phosphat-Citronensäure-Puffer, und 50 µl einer 10 mM Lösung des Substrats in Acetonitril wurden vereint und bei Raumtemperatur langsam gerührt. Zum Start der Reaktion und dann nach jeweils 15 Minuten wurden insgesamt 12 Portionen von je 10 µl einer 50 mM Wasserstoffperoxidlösung in Wasser zugegeben. 3 Stunden nach dem Start der Reaktion wurde das Reaktionsgemisch durch HPLC analysiert und der Umsatz (in Prozent) zum angegebenen Produkt, d. h. die gebildete Menge (in Prozent) an dem angegebenen Produkt, bestimmt. Die Identität der Produkte wurde durch ihre Retentionszeiten und Massenspektren (HPLC/MS) mit Hilfe von Referenzmaterialien belegt.

| Beispiel | Substrat | Peroxygenase | Produkt | Umsatz |
|---|---|---|---|---|
| 1 | Z-D,L-Phe-OH | CraUPO | H-D,L-Phe-OH | 31 % |
| 2 | Z-L-Phe-NH₂ | CraUPO | H-L-Phe-NH₂ | 59 % |
| 3 | Z-L-Ala-L-Phe-OH | CraUPO | H-L-Ala-L-Phe-OH | 20 % |
| 4 | Z-D-Ala-L-Phe-OH | CraUPO | H-D-Ala-L-Phe-OH | 15 % |
| 5 | Z-L-Arg(Mtr)-OH | CraUPO | H-L-Arg(Mtr)-OH | 76 % |
| 6 | Z-L-Arg(Mtr)-OtBu | CraUPO | H-L-Arg(Mtr)-OtBu | 62 % |
| 7 | Z-L-Arg(Tos)-OH | CraUPO | H-L-Arg(Tos)-OH | 81 % |
| 8 | Z-D-Arg(Z)₂-OH | CraUPO | Z-D-Arg(Z)-OH + Z-D-Arg-OH | 35 % +10% |
| 9 | Z-L-Asn(Xant)-OH | CraUPO | H-L-Asn(Xant)-OH | 39 % |
| 10 | Z-L-Ser(tBu)-O-4NO₂Bn | CraUPO | H-L-Ser(tBu)-O-4NO₂Bn | 6 % |
| 11 | Z-L-His(Bom)-OH | CraUPO | H-L-His(Bom)-OH | 81 % |
| 12 | Z-D,L-Phe-OH | AaeUPO | H-D,L-Phe-OH | 46 % |
| 13 | Z-L-Phe-NH₂ | AaeUPO | H-L-Phe-NH₂ | 26 % |
| 14 | Z-L-Phe-OtBu | AaeUPO | H-L-Phe-OtBu | 29 % |
| 15 | Z-L-Ala-L-Phe-OH | AaeUPO | H-L-Ala-L-Phe-OH | 16 % |
| 16 | Z-D-Ala-L-Phe-OH | AaeUPO | H-D-Ala-L-Phe-OH | 8 % |
| 17 | Z-L-Arg(Mtr)-OH | AaeUPO | H-L-Arg(Mtr)-OH | 100 % |
| 18 | Z-L-Arg(Mtr)-OtBu | AaeUPO | H-L-Arg(Mtr)-OtBu | 25 % |
| 19 | Z-D-Arg(Z)₂-OH | AaeUPO | Z-D-Arg(Z)-OH + Z-D-Arg-OH | 94 % + 3 % |
| 20 | Z-L-Asn(Xant)-OH | AaeUPO | H-L-Asn(Xant)-OH | 26 % |
| 21 | Z-L-Ser(tBu)-O-4NO₂Bn | AaeUPO | H-L-Ser(tBu)-O-4NO₂Bn | 3 % |
| 22 | Z-L-His(Bom)-OH | AaeUPO | H-L-His(Bom)-OH | 65 % |
| 23 | Z-L-Ala-L-Phe-OH | MroUPO | H-L-Ala-L-Phe-OH | 12 % |
| 24 | Z-D-Ala-L-Phe-OH | MroUPO | H-D-Ala-L-Phe-OH | 6 % |
| 25 | Z-L-Arg(Mtr)-OtBu | MroUPO | H-L-Arg(Mtr)-OtBu | 14 % |
| 26 | Z-D-Arg(Z)₂-OH | MroUPO | Z-D-Arg(Z)-OH | 12 % |
| 27 | Z-L-Asn(Xant)-OH | MroUPO | H-L-Asn(Xant)-OH | 26 % |
| 28 | Z-L-Ser(tBu)-O-4NO₂Bn | MroUPO | H-L-Ser(tBu)-O-4NO₂Bn | 3 % |
| 29 | Z-L-His(Bom)-OH | MroUPO | H-L-His(Bom)-OH | 65 % |

In den Beispielen 30 bis 41 wurde die Stabilität von substituierten Benzyloxycarbonyl-Schutzgruppen in entsprechenden N-geschützten Derivaten von H-Arg(Mtr)-OH unter standardisierten Bedingungen untersucht. 500 µl Phosphat-Citronensäure-Puffer mit pH 7 im Falle der Peroxygenasen AaeUPO und CraUPO bzw. mit pH 5.5 im Fall der Peroxygenase MroUPO, 275 µl bidestilliertes Wasser, 25 µl einer 40 mM Ascorbinsäurelösung in Wasser, 30 µl der Peroxygenaselösung mit einer Konzentration von 100 U/ml im Fall der Peroxygenasen AaeUPO und MroUPO bzw. 33.6 U/ml im Fall der Peroxygenase CraUPO in Phosphat-Citronensäure-Puffer, und 50 µl einer 10 mM Lösung des Substrats in Acetonitril wurden vereint und bei Raumtemperatur langsam gerührt. Zum Start der Reaktion und dann nach jeweils 15 Minuten wurden insgesamt 12 Portionen von je 10 µl einer 50 mM Wasserstoffperoxidlösung in Wasser zugegeben. 3 Stunden nach dem Start der Reaktion wurde das Reaktionsgemisch durch HPLC analysiert und der Umsatz (in Prozent) zum angegebenen Produkt, d. h. die gebildete Menge (in Prozent) an dem angegebenen Produkt, bestimmt. Die Identität des Produkts wurde durch seine Retentionszeit und Massenspektrum (HPLC/MS) mit Hilfe von Referenzmaterial belegt.

| Beispiel | Substrat | Peroxygenase | Produkt | Umsatz |
|---|---|---|---|---|
| 30 | 4MeOZ-L-Arg(Mtr)-OH | AaeUPO | H-L-Arg(Mtr)-OH | 100 % |
| 31 | 4MeOZ-L-Arg(Mtr)-OH | MroUPO | H-L-Arg(Mtr)-OH | 23 % |
| 32 | 4MeOZ-L-Arg(Mtr)-OH | CraUPO | H-L-Arg(Mtr)-OH | 27 % |
| 33 | 4NO₂Z-L-Arg(Mtr)-OH | AaeUPO | H-L-Arg(Mtr)-OH | 0% |
| 34 | 4NO₂Z-L-Arg(Mtr)-OH | MroUPO | H-L-Arg(Mtr)-OH | 0% |
| 35 | 4NO₂Z-L-Arg(Mtr)-OH | CraUPO | H-L-Arg(Mtr)-OH | 0% |
| 36 | 4CIZ-L-Arg(Mtr)-OH | AaeUPO | H-L-Arg(Mtr)-OH | 0% |
| 37 | 4CIZ-L-Arg(Mtr)-OH | MroUPO | H-L-Arg(Mtr)-OH | 0% |
| 38 | 4CIZ-L-Arg(Mtr)-OH | CraUPO | H-L-Arg(Mtr)-OH | 0% |
| 39 | 2NMC-L-Arg(Mtr)-OH | AaeUPO | H-L-Arg(Mtr)-OH | 0% |
| 40 | 2NMC-L-Arg(Mtr)-OH | MroUPO | H-L-Arg(Mtr)-OH | 0% |
| 41 | 2NMC-L-Arg(Mtr)-OH | CraUPO | H-L-Arg(Mtr)-OH | 0% |

Wie die unsubstituierte Z-Schutzgruppe wurde auch die N-(4-Methoxybenzyloxycarbonyl)-Schutzgruppe abgespalten. Die N-(4-Chlorbenzyloxycarbonyl)-Schutzgruppe, die N-(2-Naphthylmethyloxycarbonyl)-Schutzgruppe und die N-(4-Nitrobenzyloxycarbonyl)-Schutzgruppe waren stabil.

In den Beispielen 42 bis 65 wurde die Stabilität von verschiedenen O-Benzyl-Schutzgruppen und der O-Benzyloxycarbonyl-Schutzgruppe in Tyrosinderivaten der Formel III unter standardisierten Bedingungen untersucht. 500 µl Phosphat-Citronensäure-Puffer mit pH 7 im Falle der Peroxygenasen AaeUPO und CraUPO bzw. mit pH 5.5 im Fall der Peroxygenase MroUPO, 275 µl bidestilliertes Wasser, 25 µl einer 40 mM Ascorbinsäurelösung in Wasser, 30 µl der Peroxygenaselösung mit einer Konzentration von 100 U/ml im Fall der Peroxygenasen AaeUPO und MroUPO bzw. 33.6 U/ml im Fall der Peroxygenase CraUPO in Phosphat-Citronensäure-Puffer, und 50 µl einer 10 mM Lösung des Substrats in Acetonitril wurden vereint und bei Raumtemperatur langsam gerührt. Zum Start der Reaktion und dann nach jeweils 15 Minuten wurden insgesamt 12 Portionen von je 10 µl einer 50 mM Wasserstoffperoxidlösung in Wasser zugegeben. 3 Stunden nach dem Start der Reaktion wurde das Reaktionsgemisch durch HPLC analysiert und der Umsatz (in Prozent) zum Produkt der Formel IV, d. h. die gebildete Menge (in Prozent) an Produkt der Formel IV, durch HPLC bestimmt. Die Identität des Produkts wurde durch seine Retentionszeit und Massenspektrum (HPLC/MS) mit Hilfe von Referenzmaterial belegt.

| Beispiel | R¹⁰ im Substrat der Formel III | Peroxygenase | Umsatz zu |
|---|---|---|---|
| | 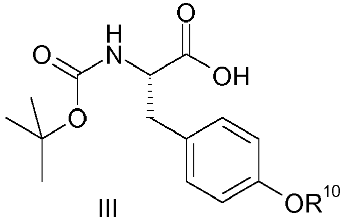 | | 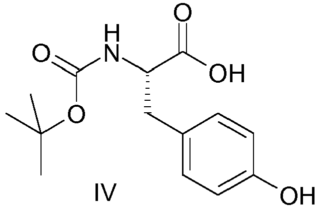 |
| 42 | 4-Methoxybenzyl | AaeUPO | 4 % |
| 43 | 4-Methoxybenzyl | MroUPO | 0 % |
| 44 | 4-Methoxybenzyl | CraUPO | 0 % |
| 45 | 3,5-Dimethoxybenzyl | AaeUPO | 2 % |
| 46 | 3,5-Dimethoxybenzyl | MroUPO | 0 % |
| 47 | 3,5-Dimethoxybenzyl | CraUPO | 2 % |
| 48 | Benzyl | AaeUPO | 4% |
| | 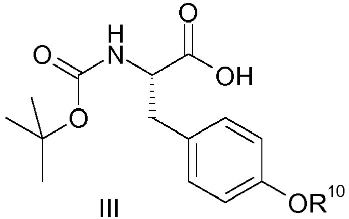 | | 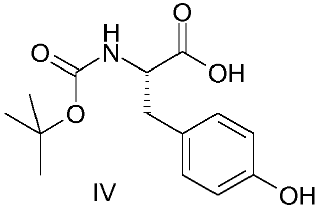 |
| 49 | Benzyl | MroUPO | 4 % |
| 50 | Benzyl | CraUPO | 7 % |
| 51 | 4-Methylbenzyl | AaeUPO | 0 % |
| 52 | 4-Methylbenzyl | MroUPO | 0 % |
| 53 | 4-Methylbenzyl | CraUPO | 0 % |
| 54 | 4-Chlorbenzyl | AaeUPO | 0 % |
| 55 | 4-Chlorbenzyl | MroUPO | 0 % |
| 56 | 4-Chlorbenzyl | CraUPO | 0 % |
| 57 | 4-Cyanbenzyl | AaeUPO | 0 % |
| 58 | 4-Cyanbenzyl | MroUPO | 0 % |
| 59 | 4-Cyanbenzyl | CraUPO | 0 % |
| 60 | 4-Nitrobenzyl | AaeUPO | 0 % |
| 61 | 4-Nitrobenzyl | MroUPO | 0 % |
| 62 | 4-Nitrobenzyl | CraUPO | 0 % |
| 63 | Benzyloxycarbonyl | AaeUPO | 10 % |
| 64 | Benzyloxycarbonyl | MroUPO | 26 % |
| 65 | Benzyloxycarbonyl | CraUPO | 35 % |

Nur im Fall der O-Benzyloxycarbonyl-Schutzgruppe und in geringerem Umfang im Fall der unsubstituierten O-Benzyl-Schutzgruppe und zum Teil der methoxy-substituierten O-Benzyl-Schutzgruppen wurde eine Abspaltung der Schutzgruppe von der aromatischen Hydroxygruppe beobachtet, die Mehrzahl der substituierten O-Benzyl-Schutzgruppen war stabil. Die N-Boc-Schutzgruppe war unter den Bedingungen der enzymatischen N-Benzyloxycarbonyl-Schutzgruppen-Abspaltung stabil, in keinem der Beispiele wurde eine Abspaltung der N-Boc-Schutzgruppe unter Bildung von Produkten mit freier Aminogruppe beobachtet.

In den Beispielen 66 bis 74 wurde die Stabilität von verschiedenen O-Benzyl-Schutzgruppen in Phenylalaninolderivaten der Formel V unter standardisierten Bedingungen untersucht. 500 µl Phosphat-Citronensäure-Puffer mit pH 7 im Falle der Peroxygenasen AaeUPO und CraUPO bzw. mit pH 5.5 im Fall der Peroxygenase MroUPO, 275 µl bidestilliertes Wasser, 25 µl einer 40 mM Ascorbinsäurelösung in Wasser, 30 µl der Peroxygenaselösung mit einer Konzentration von 100 U/ml im Fall der Peroxygenasen AaeUPO und MroUPO bzw. 33.6 U/ml im Fall der Peroxygenase CraUPO in Phosphat-Citronensäure-Puffer, und 50 µl einer 10 mM Lösung des Substrats in Acetonitril wurden vereint und bei Raumtemperatur langsam gerührt. Zum Start der Reaktion und dann nach jeweils 15 Minuten wurden insgesamt 12 Portionen von je 10 µl einer 50 mM Wasserstoffperoxidlösung in Wasser zugegeben. 3 Stunden nach dem Start der Reaktion wurde das Reaktionsgemisch durch HPLC analysiert und der Umsatz (in Prozent) zum Produkt der Formel VI, d. h. die gebildete Menge (in Prozent) an Produkt der Formel VI, bestimmt. Die Identität des Produkts wurde durch seine Retentionszeit und Massenspektrum (HPLC/MS) mit Hilfe von Referenzmaterial belegt.

| Beispiel | R¹¹ im Substrat der Formel V | Peroxygenase | Umsatz zu |
|---|---|---|---|
| | 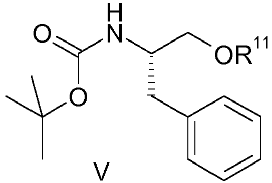 | | 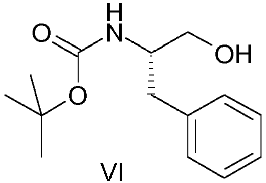 |
| 66 | 4-Methoxybenzyl | AaeUPO | 0 % |
| 67 | 4-Methoxybenzyl | MroUPO | 0 % |
| 68 | 4-Methoxybenzyl | CraUPO | 0 % |
| 69 | 3-Methoxybenzyl | AaeUPO | 0 % |
| 70 | 3-Methoxybenzyl | MroUPO | 0 % |
| 71 | 3-Methoxybenzyl | CraUPO | 0 % |
| 72 | 3,5-Dimethoxybenzyl | AaeUPO | 0 % |
| 73 | 3,5-Dimethoxybenzyl | MroUPO | 0 % |
| 74 | 3,5-Dimethoxybenzyl | CraUPO | 0 % |

In keinem der Beispiele wurde eine Abspaltung der methoxy-substituierten O-Benzyl-Schutzgruppen von der aliphatischen Hydroxygruppe beobachtet. Auch die N-Boc-Schutzgruppe war stabil, in keinem der Beispiele wurde eine Abspaltung der N-Boc-Schutzgruppe unter Bildung von Produkten mit freier Aminogruppe beobachtet.

In den Beispielen 75 bis 98 wurde die Stabilität der Fmoc-Schutzgruppe und der Aloc-Schutzgruppe in verschiedenen Substraten unter standardisierten Bedingungen untersucht. 500 µl Phosphat-Citronensäure-Puffer mit pH 7 im Falle der Peroxygenasen AaeUPO und CraUPO bzw. mit pH 5.5 im Fall der Peroxygenase MroUPO, 275 µl bidestilliertes Wasser, 25 µl einer 40 mM Ascorbinsäurelösung in Wasser, 30 µl der Peroxygenaselösung mit einer Konzentration von 100 U/ml im Fall der Peroxygenasen AaeUPO und MroUPO bzw. 33.6 U/ml im Fall der Peroxygenase CraUPO in Phosphat-Citronensäure-Puffer, und 50 µl einer 10 mM Lösung des Substrats in Acetonitril wurden vereint und bei Raumtemperatur langsam gerührt. Zum Start der Reaktion und dann nach jeweils 15 Minuten wurden insgesamt 12 Portionen von je 10 µl einer 50 mM Wasserstoffperoxidlösung in Wasser zugegeben. 3 Stunden nach dem Start der Reaktion wurde das Reaktionsgemisch durch HPLC analysiert und der Umsatz (in Prozent) zum angegebenen Produkt, d. h. die gebildete Menge (in Prozent) an dem angegebenen Produkt, bestimmt. Die Identität der Produkte wurde durch ihre Retentionszeiten und Massenspektren (HPLC/MS) mit Hilfe von Referenzmaterialien belegt.

| Beispiel | Substrat | Peroxygenase | Produkt | Umsatz |
|---|---|---|---|---|
| 75 | Fmoc-L-Phe-OH | AaeUPO | H-L-Phe-OH | 0% |
| 76 | Fmoc-L-Phe-OH | MroUPO | H-L-Phe-OH | (1) |
| 77 | Fmoc-L-Phe-OH | CraUPO | H-L-Phe-OH | 0% |
| 78 | Fmoc-L-Ser(tBu)-OH | AaeUPO | H-L-Ser(tBu)-OH | 0% |
| 79 | Fmoc-L-Ser(tBu)-OH | MroUPO | H-L-Ser(tBu)-OH | 0% |
| 80 | Fmoc-L-Ser(tBu)-OH | CraUPO | H-L-Ser(tBu)-OH | 0% |
| 81 | Fmoc-L-Thr(tBu)-OH | AaeUPO | H-L-Thr(tBu)-OH | 0% |
| 82 | Fmoc-L-Thr(tBu)-OH | MroUPO | H-L-Thr(tBu)-OH | 0% |
| 83 | Fmoc-L-Thr(tBu)-OH | CraUPO | H-L-Thr(tBu)-OH | 0% |
| 84 | Fmoc-L-Asp(tBu)-OH | AaeUPO | H-L-Asp(tBu)-OH | 0% |
| 85 | Fmoc-L-Asp(tBu)-OH | MroUPO | H-L -Asp(tBu)-OH | (2) |
| 86 | Fmoc-L-Asp(tBu)-OH | CraUPO | H-L-Asp(tBu)-OH | 0% |
| 87 | Aloc-L-Phe-OH | AaeUPO | H-L-Phe-OH | 0% |
| 88 | Aloc-L-Phe-OH | MroUPO | H-L-Phe-OH | 0% |
| 99 | Aloc-L-Phe-OH | CraUPO | H-L-Phe-OH | 0% |
| 90 | Aloc-L-Phe-NH₂ | AaeUPO | H-L-Phe-NH₂ | 0% |
| 91 | Aloc-L-Phe-NH₂ | MroUPO | H-L-Phe-NH₂ | 0% |
| 92 | Aloc-L-Phe-NH₂ | CraUPO | H-L-Phe-NH₂ | 0% |
| 93 | Aloc-L-Phe-OMe | AaeUPO | H-L-Phe-OMe | 0% |
| 94 | Aloc-L-Phe-OMe | MroUPO | H-L-Phe-OMe | 0% |
| 95 | Aloc-L-Phe-OMe | CraUPO | H-L-Phe-OMe | 0% |
| 96 | Aloc-L-Tyr-OtBu | AaeUPO | H-L-Tyr-OtBu | 0% |
| 97 | Aloc-L-Tyr-OtBu | MroUPO | H-L-Tyr-OtBu | 0% |
| 98 | Aloc-L-Tyr-OtBu | CraUPO | H-L-Tyr-OtBu | 0% |

| | | | | |
|---|---|---|---|---|
| (1) Es wurden 31 % einer ungesättigten Verbindung detektiert. (2) Es wurden 14 % einer ungesättigten Verbindung detektiert. | | | | |

Abgesehen von den Fällen der Substrate Fmoc-L-Phe-OH und Fmoc-L-Asp(tBu)-OH, in denen nicht identifizierte, vermutlich durch Hydroxylierung und Eliminierung gebildete ungesättigte Verbindungen detektiert wurden, waren die N-Fmoc-Schutzgruppe und N-Aloc-Schutzgruppe unter den Bedingungen der enzymatischen N-Benzyloxycarbonyl-Schutzgruppen-Abspaltung stabil, in keinem der Beispiele wurde eine Abspaltung der N-Fmoc-Schutzgruppe und N-Aloc-Schutzgruppe unter Bildung von Produkten mit freier Aminogruppe beobachtet.

### Beispiel 99: Benzyloxycarbonylgruppen-Abspaltung in Z-L-Phe-NH₂

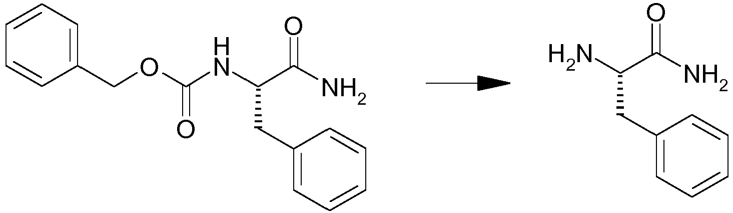

89.4 mg (0.3 mmol) Z-L-Phe-NH₂ wurden in einem 500 ml-Zweihalskolben in 10 ml Acetonitril, 50 ml Wasser und 100 ml Phosphat-Citronensäure-Puffer mit pH 7 unter Rühren bei Raumtemperatur gelöst. Anschließend wurden 2 ml einer 400 mM Ascorbinsäure-Lösung in Wasser und 300 U CraUPO (4 ml, 75 U/ml) hinzugegeben. Mit einer Geschwindigkeit von 20 ml/h erfolgte mittels Spritzenpumpe die Dosierung einer 40 mM Wasserstoffperoxid-Lösung zu dem Ansatz. Nach 5 Stunden wurde die Dosierung umgestellt auf eine 200 mM Wasserstoffperoxid-Lösung und eine Dosiergeschwindigkeit von 4 ml/h. Zusätzlich wurden weitere 2 ml wässrige Ascorbinsäure-Lösung zugegeben. In regelmäßigen Abständen wurden Proben von jeweils 75 µl entnommen, zu 75 µl Acetonitril und 10 µl 10 mM Natriumazidlösung in Wasser pipettiert und durch HPLC analysiert. Nach einer Reaktionszeit von 24 Stunden wurde die Zufuhr von Wasserstoffperoxid eingestellt und das Gemisch ultrafiltriert (Amicon 8200), mit 37 %iger Natronlauge auf einen pH-Wert von 10 eingestellt und zunächst mehrfach mit Essigsäureethylester und dann mit n-Butanol extrahiert. Nach Entfernen des Lösungsmittels aus den vereinigten Extrakten im Vakuum blieben 80 mg eines weiß-bräunlichen Feststoffs zurück, der in Methanol aufgenommen und durch Chromatographie an Kieselgel mit Dichlormethan/Methanol (9:1) gereinigt wurde. Ausbeute: 37.8 mg (76.7 %) H-L-Phe-NH₂; Reinheit: 93.1 % (UV, 210 nm).
Schmelzpunkt: 92-94 °C.
¹H-NMR (300 MHz, D₆-DMSO): δ (ppm): 2.57 (dd, J = 13.2 Hz und 8.5 Hz, 1H, CH₂), 2.90 (dd, J = 13.2 Hz und 4.5 Hz, 1H, CH₂), 3.29 (dd, J = 8.5 Hz und 4.5 Hz, 1H, CH), 7.19-7.29 (m, 5H, Phenyl).

### Beispiel 100: Benzyloxycarbonylgruppen-Abspaltung in Z-L-Arg(Mtr)-OH

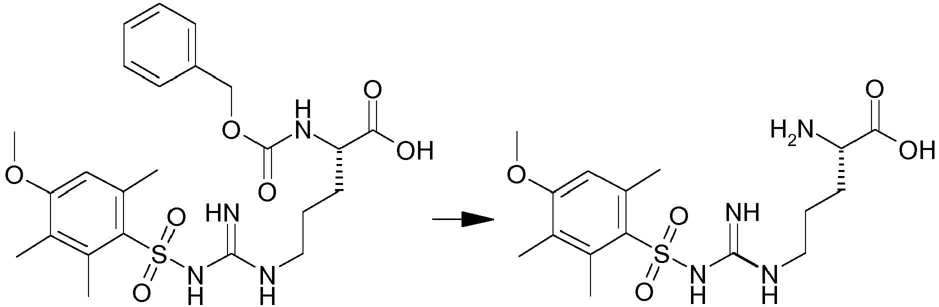

124 mg (0.2 mmol) Z-L-Arg(Mtr)-OH-Cyclohexylammoniumsalz wurden in 10 ml Acetonitril, 40 ml Wasser und 100 ml Phosphat-Citronensäure-Puffer mit pH 7 unter Rühren bei Raumtemperatur gelöst. Anschließend wurden 2 ml einer 400 mM Ascorbinsäure-Lösung in Wasser und 300 U CraUPO (4 ml, 75 U/ml) hinzugegeben. Mittels Spritzenpumpe erfolgte die Dosierung einer 40 mM Wasserstoffperoxid-Lösung zu dem Ansatz mit einer Dosiergeschwindigkeit von 20 ml/h. Für die HPLC-Analytik wurden 75 µl-Proben entnommen und zu 75 µl Acetonitril und 10 µl 10 mM Natriumazidlösung in Wasser pipettiert. Nach einer Reaktionszeit von 4 Stunden wurde die Zufuhr von Wasserstoffperoxid eingestellt und das Gemisch ultrafiltriert (Amicon 8200). Das Filtrat (ca. 250 ml) wurden dreimal mit jeweils 20 ml n-Butanol extrahiert, die vereinigten organischen Phasen mit Natriumsulfat getrocknet und das Lösungsmittel im Rotationsverdampfer abdestilliert. Der Rückstand wurde in 50 %igem wässrigem Acetonitril aufgenommen und durch präparative HPLC gereinigt. Das braune harzige Produkt kristallisierte in Dichlormethan. Ausbeute: 70.9 mg (91.7 %) H-L-Arg(Mtr)-OH; Reinheit: 98.0 % (UV, 210 nm).
Schmelzpunkt: 158-161 °C.
¹H-NMR (300 MHz, CD₃OD): δ (ppm): 1.69-1.73 (m, 2H, CH₂-C-C-C=O), 1.82-1.96 (m, 2H, CH₂-C-C=O), 2.15 (s, 3H, C-CH₃), 2.63 (s, 3H, C-CH₃), 2.69 (s, 3H, C-CH₃), 3.20-3.27 (m, 2H, N-CH₂), 3.86 (s, 3H, O-CH₃), 4.01 (t, J = 6.1 Hz, 1H, CH), 6.70 (s, 1H, Phenyl).

### Beispiel 101: Benzyloxycarbonylgruppenabspaltung in Z-L-His(Bom)-OH

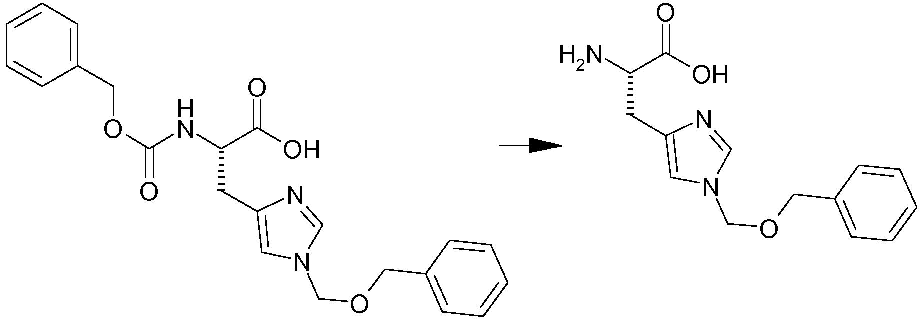

82 mg (0.2 mmol) Z-L-His(Bom)-OH wurden in einem 500 ml-Kolben in 10 ml Acetonitril, 50 ml Wasser und 100 ml Phosphat-Citronensäure-Puffer mit pH 7 unter Rühren bei Raumtemperatur gelöst. Anschließend wurden 2 ml einer 400 mM Ascorbinsäure-Lösung in Wasser und 600 U CraUPO (8 ml, 75 U/ml) hinzugegeben. Mittels Spritzenpumpe erfolgte die Dosierung einer 40 mM wässrigen Wasserstoffperoxid-Lösung mit einer Dosiergeschwindigkeit von 10 ml/h. Nach 7 Stunden wurde die Dosierung umgestellt auf eine Dosiergeschwindigkeit von 4 ml/h. Vor Dosierungsbeginn sowie alle 30 Minuten nach Reaktionsstart, ab 2 Stunden stündlich, wurden 75 µl-Proben entnommen und zu 75 µl Acetonitril und 10 µl 10 mM Natriumazidlösung in Wasser pipettiert und durch HPLC analysiert. Z-L-His(Bom)-OH war nach 24 Stunden Reaktionszeit zu 85 % zu H-L-His(Bom)-OH umgesetzt. Das Reaktionsgemisch (ca. 300 ml) wurden zunächst mit Essigsäureethylester und dann mit n-Butanol extrahiert. Da nur ein geringer Teil des Produktes in die organische Phase extrahiert wurde, wurde anschließend eine kontinuierliche Flüssig-Flüssig-Extraktion im Perforator über 7 Stunden mit 500 ml n-Butanol im Vorlagekolben bei einer Heiztemperatur von 170 °C durchgeführt. Es wurden dabei ca. 90 % des H-L-His(Bom)-OH aus der wässrigen Phase extrahiert. Nach Entfernen des n-Butanols wurde der Rückstand mit Methanol digeriert und filtriert. Das im Methanol gelöste H-L-His(Bom)-OH wurde durch Chromatographie an Kieselgel mit Dichlormethan/Methanol//Essigsäure (6:4:1) gereinigt. Ausbeute: 8.8 mg (16.0 %) H-L-His(Bom)-OH; Reinheit: 88.0 % (UV, 210 nm).
¹H-NMR (300 MHz, D₆-DMSO): δ (ppm): 2.72 (dd, 1H, CH₂-C-C=O), 2.81 (dd, 1H, CH₂-C-C=O), 4.28 (m, 1H, CH), 4.42 (s, 2H, O-CH₂-C), 5.40 (d, 1H, N-CH₂-O), 5.49 (d, 1H, N-CH₂-O), 6.89 (s, 1H, Imidazol), 7.21-7.32 (m, 5H, Phenyl), 7.74 (s, 1H, Imidazol).

## Patentansprüche

1. Verfahren zur Abspaltung von Benzyloxycarbonyl-Schutzgruppen, **dadurch gekennzeichnet, dass** eine N-Benzyloxycarbonyl-Verbindung oder eine N-(4-Methoxybenzyloxycarbonyl)-Verbindung in einem wasserhaltigen Lösungsmittel in Gegenwart einer unspezifischen Peroxygenase aus den Basidiomyceten Agrocybe aegerita, Coprinellus radians oder Marasmius rotula mit Wasserstoffperoxid umgesetzt wird.

2. Verfahren gemäß Anspruch 1, **dadurch gekennzeichnet, dass** es bei einem pH-Wert von 4 bis 9 durchgeführt wird.

3. Verfahren gemäß einem oder mehreren der Ansprüche 1 und 2, **dadurch gekennzeichnet, dass** es in Gegenwart eines Puffers durchgeführt wird.

4. Verfahren gemäß einem oder mehreren der Ansprüche 1 bis 3, **dadurch gekennzeichnet, dass** es bei 10 °C bis 70 °C durchgeführt wird.

5. Verfahren gemäß einem oder mehreren der Ansprüche 1 bis 4, **dadurch gekennzeichnet, dass** das wasserhaltige Lösungsmittel ein Gemisch aus Wasser und einem oder mehreren organischen Lösungsmitteln ist.

6. Verfahren gemäß einem oder mehreren der Ansprüche 1 bis 5, **dadurch gekennzeichnet, dass** es in Gegenwart eines Radikalfängers durchgeführt wird.

7. Verfahren gemäß einem oder mehreren der Ansprüche 1 bis 6, **dadurch gekennzeichnet, dass** Wasserstoffperoxid sukzessive zudosiert wird.

8. Verfahren gemäß einem oder mehreren der Ansprüche 1 bis 7, **dadurch gekennzeichnet, dass** eine N-Benzyloxycarbonyl-Verbindung umgesetzt wird.

9. Verfahren gemäß einem oder mehreren der Ansprüche 1 bis 8, **dadurch gekennzeichnet, dass** ein N-Benzyloxycarbonyl-aminosäurederivat oder N-Benzyloxycarbonyl-peptidderivat umgesetzt wird.

10. Verwendung einer unspezischen Peroxygenase aus den Basidiomyceten Agrocybe aegerita, Coprinellus radians oder Marasmius rotula zur Abspaltung von Benzyloxycarbonyl-Schutzgruppen durch Umsetzung einer N-Benzyloxycarbonyl-Verbindung oder einer N-(4-Methoxybenzyloxycarbonyl)-Verbindung mit Wasserstoffperoxid in einem wasserhaltigen Lösungsmittel.

## Claims

1. Process for the cleavage of benzyloxycarbonyl protective groups, **characterized in that** an N-benzyloxycarbonyl compound or an N-(4-methoxybenzyloxycarbonyl) compound is reacted with hydrogen peroxide in a solvent comprising water in the presence of an unspecific peroxygenase from the Basidiomycetes Agrocybe aegerita, Coprinellus radians or Marasmius rotula.

2. Process according to Claim 1, **characterized in that** said process is carried out at a pH of 4 to 9.

3. Process according to one or more of Claims 1 and 2, **characterized in that** said process is carried out in the presence of a buffer.

4. Process according to one or more of Claims 1 to 3, **characterized in that** said process is carried out at 10°C to 70°C.

5. Process according to one or more of Claims 1 to 4, **characterized in that** the solvent comprising water is a mixture of water and one or more organic solvents.

6. Process according to one or more of Claims 1 to 5, **characterized in that** said process is carried out in the presence of a free radical scavenger.

7. Process according to one or more of Claims 1 to 6, **characterized in that** hydrogen peroxide is metered in gradually.

8. Process according to one or more of Claims 1 to 7, **characterized in that** an N-benzyloxycarbonyl compound is reacted.

9. Process according to one or more of Claims 1 to 8, **characterized in that** an N-benzyloxycarbonyl amino acid derivative or N-benzyloxycarbonyl peptide derivative is reacted.

10. Use of an unspecific peroxygenase from the Basidiomycetes Agrocybe aegerita, Coprinellus radians or Marasmius rotula for the cleavage of benzyloxycarbonyl protective groups by reacting an N-benzyloxycarbonyl compound or an N-(4-methoxybenzyloxycarbonyl) compound with hydrogen peroxide in a solvent comprising water.

## Revendications

1. Procédé de clivage de groupes protecteurs benzyloxycarbonyle, **caractérisé en ce qu'**un composé de N-benzyloxycarbonyle ou un composé de N-(4-méthoxybenzyloxycarbonyle) est mis en réaction avec du peroxyde d'hydrogène dans un solvant contenant de l'eau en présence d'une peroxygénase non spécifique des basidiomycètes Agrocybe aegerita, Coprinellus radians ou Marasmius rotula.

2. Procédé selon la revendication 1, **caractérisé en ce qu'**il est réalisé à un pH de 4 à 9.

3. Procédé selon une ou plusieurs des revendications 1 et 2, **caractérisé en ce qu'**il est réalisé en présence d'un tampon.

4. Procédé selon une ou plusieurs des revendications 1 à 3, **caractérisé en ce qu'**il est réalisé à une température de 10 °C à 70 °C.

5. Procédé selon une ou plusieurs des revendications 1 à 4, **caractérisé en ce que** le solvant contenant de l'eau est un mélange d'eau et d'un ou de plusieurs solvants organiques.

6. Procédé selon une ou plusieurs des revendications 1 à 5, **caractérisé en ce qu'**il est réalisé en présence d'un capteur de radicaux.

7. Procédé selon une ou plusieurs des revendications 1 à 6, **caractérisé en ce que** du peroxyde d'hydrogène est ajouté successivement.

8. Procédé selon une ou plusieurs des revendications 1 à 7, **caractérisé en ce qu'**un composé de N-benzyloxycarbonyle est mis en réaction.

9. Procédé selon une ou plusieurs des revendications 1 à 8, **caractérisé en ce qu'**un dérivé d'acide N-benzyloxycarbonyl-aminé ou un dérivé de N-benzyloxycarbonyl-peptide est mis en réaction.

10. Utilisation d'une peroxygénase non spécifique des basidiomycètes Agrocybe aegerita, Coprinellus radians ou Marasmius rotula pour le clivage de groupes protecteurs benzyloxycarbonyle par mise en réaction d'un composé de N-benzyloxycarbonyle ou d'un composé de N-(4-méthoxybenzyloxycarbonyle) avec du peroxyde d'hydrogène dans un solvant contenant de l'eau.
